# EUROPEAN PATENT SPECIFICATION

(11) **EP 4 013 315 B1**
(45) Date of publication and mention of the grant of the patent: **26.02.2025**
(21) Application number: 21725513.2
(22) Date of filing: 12.05.2021
(51) Int. Cl.: A61B 17/06

(54) **NEEDLE HOLDER AND PACKAGE FOR SURGICAL SUTURE MATERIAL**
NADELHALTER UND VERPACKUNG FÜR CHIRURGISCHES NAHTMATERIAL
SUPPORT D'AIGUILLE ET EMBALLAGE POUR SUTURE CHIRURGICALE

(30) Priority: 14.05.2020 EP 20382404
(43) Date of publication of application: 22.06.2022
(73) Proprietor: B. Braun Surgical S.A., 08191 Rubí (ES)
(72) Inventor: ARANDA GARCÍA, José Antonio, 08191 Rubí (ES); PINO CARDOSO, Antonio, 08191 Rubí (ES)
(74) Representative: Patentanwälte Ruff, Wilhelm, Beier, Dauster & Partner mbB
(86) International application number: PCT/EP2021/062662
(87) International publication number: WO 2021/228962

(56) References cited:
- EP-A1- 0 755 657
- EP-A1- 2 153 780
- EP-A1- 3 398 524
- EP-A2- 1 842 494
- WO-A1-97/41780
- DE-A1- 102010 055 049
- US-A- 5 213 210

## Description

### TECHNICAL FIELD AND PRIOR ART

The invention relates to a needle holder for a package for surgical suture material and to a package for surgical suture material having a needle holder.

It is known to retain surgical suture material having needles affixed at one or both ends in packages, also referred to as suture packages. Those packages typically are provided with a winding channel extending along an oval-shaped, in particular a stadium-shaped (rectangular with semi-circular ends) path in which the suture material is inserted. Further, these packages comprise at least one needle holder or needle park for holding the needle(s).

EP 2 153 780 A1 discloses a package for surgical suture material having at least one needle affixed thereto, the package comprising a base member with at least one needle holder. The needle holder comprises a resilient tension element in the formed of an arm and a bearing serving as a counterbearing, which is connected fixedly to the base member. The arm has two ends, wherein a first end is connected fixedly to the base member and a second end is free, so that the arm is pivotable in a pivoting plane parallel to the base member. At the second end, a stop is provided. The second end of the arm in a neutral, undeformed state of the arm is arranged next to the bearing with a back side of the stop facing the bearing. For clamping a needle, the arm is resiliently deformed and the second end is moved across the bearing such that a front side of the stop faces the bearing, wherein the needle can be clamped between the bearing and the stop.

US 5,213,210 A1 shows a package for surgical suture material with a base and a needle park comprising two cantilevered arms, which extend horizontally above the base.

WO 97/41780 A1 shows a package for surgical suture material with a needle support structure including a relatively inflexible leg having a notch thereon for supporting a first portion of the surgical needle and one or two relatively flexible legs for engaging and biasing the surgical needle within the notch at second and third portions of the surgical needle.

EP 1 842 494 A2 shows a package for surgical suture material with a cover having needle parks on the outer surface thereof, which needle park includes three cantilevered members that provide a three-point contact with the needle.

### SUMMARY OF THE INVENTION

It is the object of the present invention to provide a needle holder for a package for surgical suture material ensuring a secure holding of a needle. It is further the object of the present invention to provide a package for surgical suture material having a needle holder.

According to a first aspect, a needle holder for securing a needle in a package for surgical suture material is provided, the needle holder having a nose protruding from a base plane, and two arms, which are resiliently deformable and/or displaceable in a plane parallel to the base plane, wherein the arms are each provided with a stop for contacting the needle, wherein the stops are arranged at opposite sides of the nose, wherein a front end of the nose is serving as a counterbearing for the needle between the two stops, wherein the arms are adapted for forcing the needle contacted by the stops against the front end of the nose, wherein the arms are L-shaped comprising a pivotally mounted lever with a first end serving as a pivot mount, and a second end, wherein in a plane parallel to the base plane the stop protrudes from the second end towards the nose, and wherein in a neutral initial position the levers of the arms extend perpendicular to the nose and the stops extend parallel to the nose. The two stops and the nose contact the needle in three contact regions, ideally in three contact points, thus allowing for a secure clamping of the needle in the needle holder.

The arms and the nose in one embodiment are formed integrally with a base member of a package, wherein a top surface of the base member forms the base plane. In other embodiments, the needle holder is formed separately and mounted to the base member. The arms are pivotally mounted, wherein the arms are pivotable in a pivoting plane parallel to the base plane.

The arms are L-shaped comprising a pivotally mounted lever with a first end serving as a pivot mount, and a second end, wherein in a plane parallel to the base plane the stop protrudes from the second end towards the nose. In the context of the application, the expressions "first", and "second" is not to be interpreted as defining a serial or numerical limitation but instead is only used to distinguish or identify various members of a group. A pivot mount in one embodiment is achieved by a flexure bearing between the base plane and the arm formed integrally with the base plane.

An optimal arrangement of the arms with respect to the nose can be chosen by the skilled person among others in consideration of a form of a needle to be clamped. For example, a position may be chosen different for a straight needle than for bent needles.

The arms are arranged such that in neutral initial position the levers of the arms extend perpendicular to the nose and the stops extend in parallel to the nose. This arrangement allows for a secure clamping of a curved needle by pivoting the levers against restoration forces out of the neutral initial position.

In embodiments, the arms are mirror-symmetrically arranged in relation to the nose, wherein in particular the arms are identical in design. The mirror-symmetric arrangement ensures equal forces to be applied by both arms without the need for complex calculations or tests when designing the arms of the needle holder.

In one embodiment, the nose is provided on a rib having a slot for receiving the needle. This design facilitates an insertion of the needle into the needle holder in manufacturing.

According to a second aspect, a package for surgical suture material is provided, the package comprising a base member with at least one needle holder as described above. The needle holder can be advantageously combined with different types of packages, for example with a package described in EP 2 153 780 A1. In embodiments of the invention, the base member is formed integrally with the needle holder for example by injection molding, wherein in particular the nose and the arms are formed integrally with the base member. In this case, for example flexure bearings are provided for pivotally connecting the arms at the second ends to a top surface of the base member.

In one embodiment, the package further comprises a base member and a cover member, wherein the base member has a plurality of pins protruding from a top surface and the cover member has pin receiving holes, which pin receiving holes are adapted for receiving the pins of the base member for mounting the cover member to the base member. A connection of the base member and the cover member is chosen by the skilled person considering among others a material of the base member and the cover member. In one embodiment, the base member and the cover member are both made of plastic material, wherein the cover member is welded to the base member for example applying ultrasonic welding technologies. In other embodiments, the cover member is secured to the base member using a snap-in fixture. For this purpose, the pins in one embodiment are provided with a head, which is larger in diameter than the pin receiving holes. In still another embodiment, a rivet fixture is provided, wherein pins, which not necessarily have a larger diameter head, are provided, which pins are deformed after placing the cover member on the base member for securing the cover member to the base member. A snap-in fixture or a rivet fixture are advantageous as such a fixture imposes less restriction on the choice of raw materials used for manufacturing the base member or the cover member. The height of the pins is chosen such that after a connection of the cover member and the base member the pins do not or only marginally protrude from a top surface of the package. In particular when fixing the cover member to the base member using a rivet fixture, distal ends of the pins are deformed such that a head having a larger diameter is formed and the pins are shorter in height than before the deformation. In this case, after a connection of the cover member and the base member only the larger diameter heads of the pins protrude from a top surface of the package.

In one embodiment, the cover member is provided with a plurality of guiding holes, which guiding holes are arranged between the pin receiving holes and an outer circumference of the cover member and are adapted for receiving winding pins of a winding apparatus, wherein at least one of the pin receiving holes forms as a common opening together with a neighboring guiding hole. The guiding holes are adapted for receiving winding pins of a winding head of a winding apparatus as described for example in EP 3 438 029 A1. Providing a common opening for the guiding holes and the pin receiving holes allows optimizing an area of the cover member, which is occupied by guiding holes and/or pin receiving holes. In one embodiment, the winding pins are received in the common opening during manufacturing, in particular during winding of the suture material, wherein after the winding is completed the winding pins are withdrawn from the guiding holes and only after the withdrawal of the winding pins, the pins of the base member are inserted. In other embodiments, a guiding hole and a pin receiving hole are merged for forming one common opening, which is larger in size than either one of the guiding hole and pin receiving hole taken separately. The cover member having at least one common opening for receiving a winding pin and/or a pin of a base member is also advantageous for other packages for surgical suture material than the one described above, for examples packages not having a needle holder or packages provided with different needle holders, including for example, but not limited to, needle holders described in EP 2 153 780 A1. When providing larger size common openings, the cover member can be mounted to the base member before a winding of the suture material, wherein the cover member is slightly lifted and deflected for an introduction of the suture material into the channel.

In one embodiment, the pin receiving holes and the guiding holes in each case are arranged along an oval path having semi-circular ends, wherein the pin receiving holes arranged in the region of the semi-circular ends at least partly form common openings together with the respectively associated neighboring guiding holes. The shape of the oval path is in particular in embodiments of the invention geometrically similar to the basic shape of the package.

In one embodiment, the pin receiving holes arranged in the region of the semi-circular ends are at least partly arranged radially inward of the respectively associated neighboring guiding holes of a common opening. Thereby, a distance between a pin receiving hole and a guiding hole of a common opening is minimized, which allows to maximize the unperforated or punctured surface of the cover member and to improve a stability of the cover member.

In one embodiment, a radius of a first semi-circular end of the oval path of the pin receiving holes is larger than a radius of a second semi-circular end of the oval path of the pin receiving holes, wherein in particular the pin receiving holes arranged at the first semi-circular end partly overlap with the associated neighboring guiding holes for forming the common openings and the pin receiving holes arranged at the second semi-circular end are connected to the associated neighboring guiding holes via bridges for forming the common openings. In one embodiment, the pin receiving holes and the guiding holes in each case have a circular basic shape. In other embodiments, at least one of the pin receiving holes and the guiding holes has a non-circular basic shape.

In one embodiment, upon winding a suture material to the package, winding pins are inserted from above through the guiding holes of the cover member, wherein distal ends of the winding pins contact a top surface of the base member, thereby forcing the base member away from the cover member for forming or increasing a gap between the cover member and the base member. In another embodiment, the base member is provided with a plurality of guiding holes arranged in alignment with the guiding holes of the cover member. Hence, winding pins of a winding apparatus can be inserted through the guiding holes of the cover member and/or the base member from above or below the package.

In one embodiment, the pins protruding from a top surface of the base member are arranged along a separation wall protruding from the top surface of the base member, which separation wall separates a winding channel of the package from a center area. In one embodiment, a height of the separation wall defines a distance between the cover member and the base member, wherein the separation wall delimits a winding channel of the package from a center area.

In one embodiment, the needle holder is provided at the center area, wherein the separation wall has an opening permitting a passage of the suture material. In particular, in one embodiment, a needle holder is accessible by a user from a cut-out in the cover member for accessing the needle holder and removing the needle clamped in the needle holder, wherein upon removing the needle a suture material attached thereto is pulled out the winding channel though the opening in the separation wall.

In one embodiment, the base member and the cover member contact each other at their outer circumferences. In one embodiment, the base member and the cover member at their outer circumferences are provided with interlocking teeth and notches. The teeth and notches provide a barrier to hinder a suture material retained in a winding channel between the base member and the cover member from getting out of the winding channel.

A material of the cover member and the base member can be chosen by a skilled person considering health regulations, environmental compatibilities and/or other restrictions. In one embodiment, the cover member is made from paper material. Paper material is environmental compatible and has advantageous absorbent properties.

### BRIEF DESCRIPTION OF THE DRAWINGS

In the following, an embodiment of the invention will be described in detail with reference to the drawings. Throughout the drawings, the same elements will be denoted by the same reference numerals. The drawings schematically show
- Fig. 1: in a top view an embodiment of a package for surgical suture material comprising a cover member and a base member,
- Fig. 2: in a top view the cover member of the package of Fig. 1,
- Fig. 3: in a top view the base member of the package Fig. 1,
- Fig. 4: in a side view the base member of Fig. 3, and
- Fig. 5: a detail V of Fig. 3.

### DETAILED DESCRIPTION OF EMBODIMENTS OF THE INVENTION

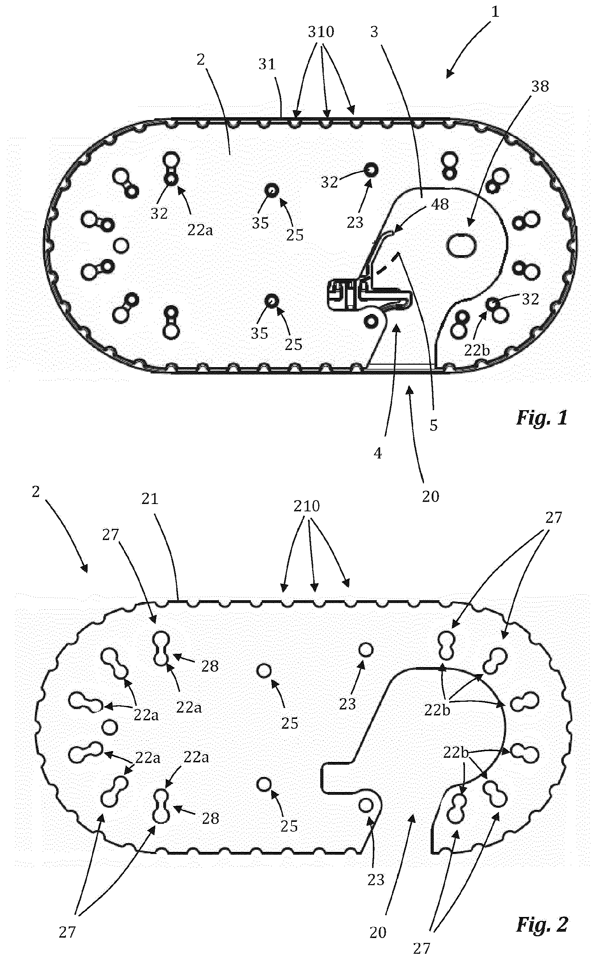

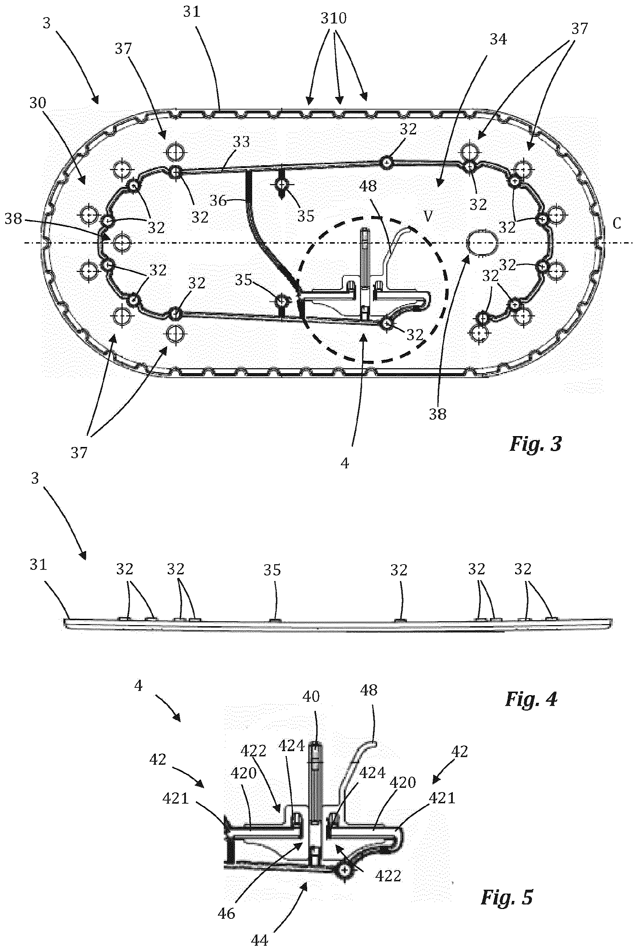

Fig. 1 shows in a top view a package 1 for surgical suture material (not shown) comprising a cover member 2 and a base member 3. The cover member 2 is shown in isolation in Fig. 2. The base member is shown in isolation in Figs. 3 and 4.

In the embodiment shown, the cover member 2 and the base member 3 both have an oval shape. More particular, in the embodiment shown, the cover member 2 and the base member 3 both are stadium-shaped, i.e. in the shape of a rectangular with two opposing semi-circular ends.

The cover member 2 is attached to the base member 3, wherein a winding channel 30 (see Fig. 3) is formed between the cover member 2 and the base member 3. The cover member 2 and the base member 3 are at least essentially congruent. More particular, in the embodiment shown, the base member 3 is provided at its outer circumference with a rim 31 and the cover member 2 is inserted into a space defined by the rim 31. The rim 31 has inwardly protruding teeth 310, which engage or interlock with notches 210 provided at an outer circumference 21 of the cover member 2. Hence, the rim 31 confines the winding channel 30 for retaining a suture material inside the winding channel 30.

The base member 3 is provided with a needle holder 4, which is shown in more detail in Fig. 5.

The needle holder 4 shown in the figures comprises a nose 40 and two L-shaped arms 42, which are arranged at opposite sides of the nose 40. In the embodiment shown, the arms 42 are mirror-symmetrically arranged in relation to the nose 40. The arms 42 are resiliently deformable out of a neutral initial position shown in Fig. 5 in a plane parallel to the drawing plane, i.e. in a plane parallel to a top surface of the base member 3.

Each arm 42 comprises a lever 420 and a stop 424. In the neutral initial position shown in Fig. 5, the levers 420 are arranged so as to extend perpendicular to the nose 40. A first end 421 of each of the levers 420, which is facing away from the nose 40, is connected to the top surface of the base member 3. The connection in one embodiment is achieved by forming the arms 42 integrally with the base member 3 wherein a flexure bearing is provided between each arm 42 and the base member 3. The first ends 421 serve as a pivot mount for the levers 420, which are pivotable about an axis perpendicular to the top surface of the base member 3 about the pivot mount.

A stop 424 is provided at a second end 422 of each lever 420, which stop 424 in the embodiment shown protrudes from the lever 420 in a direction parallel to the top surface of the base member 3 and extends in parallel to the nose 40 towards the nose 40. The stops 424 of the two arms 42 are arranged at opposite sides of the nose 40. Upon deformation of the arms 42 out of the neutral initial position shown in Fig. 5, a restoration force will force a needle contacted by the stops against a front end of the nose 40, which front end is serving as a counterbearing for the needle. This allows to securely clamp a needle between the stops 424 and the nose 40.

In the embodiment shown, the nose 40 is provided on a rib 44 having a slot 46 for receiving a needle. In other words, a needle can be inserted in the slot 46 and forced against the nose40 by means of the arms 42.

In Fig. 1, a needle 5 retained in the needle holder 4 is schematically shown by a dotted line. For removing the needle 5 from the needle holder 4, the needle 5 is accessible via a cut-out 20 in the cover member 2. Adjacent to the needle holder 4, the base member 3 is provided with a slit 48 for easing a deformation of a region of the base member 3 when grasping the needle 5.

The needle holder 4 can be combined with different types of suture packages. In the embodiment shown, the cover member 2 is fixed to the base member 3 using a pin connection, which pin connection is described in more detail in the following. The pin connection described below is also advantageous for other types of packages for surgical suture material with or without a needle holder 4.

The base member 3 (shown in detail in Figs. 3 and 4) is provided with a plurality of pins 32 which are protruding from a top surface of the base member 3. As best seen in Fig. 4, in the embodiment shown, the pins 32 extend beyond an upper end of the rim 31. The pins 32 are arranged along a separation wall 33, which separation wall 33 also protrudes from the top surface of the base member 3. The separation wall 33 separates the winding channel 30 from a center area 34 of the base member 3. In the embodiment shown, two additional pins 35 are provided, which are arranged inside of the separation wall 33. In addition, in the embodiment shown a transverse wall 36 is provided, which extends between two long sides of the separation wall 33 across the center area 34. The needle holder 4 is arranged in the center area 34, wherein the separation wall 33 has an opening permitting a passage of the suture material between the winding channel 30 on the outside of the separation wall 33 and the needle holder 4.

As best seen in Fig. 3, the base member 3 is further provided with a plurality of guiding holes 37, which are adapted for receiving winding pins (not shown) of a winding head of a winding apparatus as described for example in EP 3 438 029 A1. The guiding holes 37 are arranged outwardly of the separation wall 33. In addition, two central guiding holes 38 are arranged inwardly of the separation wall 33 along a center line of the base member 3. In the embodiment shown, the central guiding hole 38 provided at a right side in Fig. 3, which is arranged closer to the needle holder 4 is a formed as an oblong hole. All other guiding holes 37, 38 have a circular basic shape.

The cover member 2 is provided with a plurality of pin receiving holes 22a, 22b, 23, 25, which pin receiving holes 22a, 22b, 23, 25 are adapted for receiving the pins 32, 35 protruding from the top surface of the base member 3 when inserting the cover member 2 inside the rim 31 of the base member 3. In the embodiment shown, a rivet fixture is provided, wherein the pins 33, 35 are inserted in the pin receiving holes 22a, 22b, 23, 25 and distal ends of the pins 33, 35 are deformed after the insertion for a form-locking, in particular using heat created by ultrasonic energy. Thereby a reliable connection is achieved. In alternative embodiments, the pins 33, 35 are glued or welded to the cover member 2.

The cover member 2 is further provided with a plurality of guiding holes 27, which guiding holes are arranged between the pin receiving holes 22a, 22b and the outer circumference 21 of the cover member 2. The guiding holes 27 are adapted for receiving the winding pins (not shown) of the winding head of the winding apparatus as described for example in EP 3 438 029 A1.

A first set of pin receiving holes 22a, 22b, 23 is arranged along an oval path having semi-circular ends. The oval path is congruent to a curvature of the separation wall 33 of the base member 3. Two additional pin receiving holes 25 are arranged inwardly of the oval path, which upon mounting the cover member 2 to the base member 3 receive the additional pins 35 arranged inwardly of the separation wall 33.

The guiding holes 27 of the cover member 2 are also arranged along an oval path having semi-circular ends. In the embodiment shown, the pin receiving holes 22a, 22b arranged in the region of the semi-circular ends are at least essentially arranged radially inward of the respectively associated neighboring guiding holes 27. Further, in the embodiment shown, all pin receiving holes 22a, 22b arranged in the region of the semi-circular ends of the oval path in which the first set of pin receiving holes 22a, 22b, 23 is arranged form common openings together with the respectively associated neighboring guiding holes 27. Providing common openings, which function as pin receiving holes 22a, 22b and guiding holes 27 is advantageous for optimizing a perforated area of the cover member 2. This allows for example to increase a stability of a cover member 2, in particular when made of a paper material, and/or to maximize an area used for printing.

In the embodiment shown, the semi-circular ends of the oval path along which the guiding holes 27 are arranged have the same radius.

However, a radius of a first semi-circular end of the oval path of the pin receiving holes 22a, 22b, 23, which is shown on the right in Fig. 1 and 2, and along which pin receiving holes 22b are arranged, is larger than a radius of a second semi-circular end of the oval path of the pin receiving holes 22a, 22b, 23 shown, which is shown on the left in Fig. 1 and 2, and along which pin receiving holes 22a are arranged. Hence, the pin receiving holes 22b arranged at the first semi-circular end are located closer to the respectively associated neighboring guiding holes 27 than pin receiving holes 22a arranged at the second semi-circular end. More particular, in the embodiment shown, the pin receiving holes 22b arranged at the first semi-circular end partly overlap with the associated neighboring guiding holes 27 for forming the common openings and the pin receiving holes 22a arranged at the second semi-circular end are connected to the associated neighboring guiding holes 27 via bridges 28 for forming the common openings.

In the embodiment shown, the pin receiving holes 22a, 22b and the guiding holes 27 in each case have a circular basic shape. Hence, the common openings formed by the receiving holes 22a arranged along the second semi-circular end and the associated neighboring guiding holes 27 are dumbbell-shaped. The common openings formed by the receiving holes 22b arranged along the first semi-circular end and the associated neighboring guiding holes 27 are figure-of-eight- shaped. Alternative shapes are conceivable to the skilled person.

The cover member 2 has a cut-out 20 allowing access to the needle holder 4 after the cover member 2 is mounted to the base member 3. In the embodiment shown, the pin receiving hole 22b arranged along the first semi-circular end and the pin receiving hole 22a arranged along the second semi-circular end in each case are equidistantly arranged with the exception of the pin receiving hole 22b on the first semi-circular end close to a border of the cut-off 20, which is displaced for avoiding an interference with the cut-off.

## Claims

1. A needle holder (4) for securing a needle in a package for surgical suture material, the needle holder (4) having a nose (40) protruding from a base plane, and two arms (42), which are resiliently deformable and/or displaceable in a plane parallel to the base plane, wherein the arms (42) are each provided with a stop (424) for contacting the needle, wherein the stops (424) are arranged at opposite sides of the nose (40), wherein a front end of the nose (40) is serving as a counterbearing for the needle between the two stops (424), and wherein the arms (42) are adapted for forcing the needle contacted by the stops (424) against the front end of the nose (40), wherein the arms (42) are L-shaped comprising a pivotally mounted lever (420) with a first end (421) serving as a pivot mount, and a second end (422), wherein in a plane parallel to the base plane the stop (424) protrudes from the second end (422) towards the nose (40), **characterized in that**
in a neutral initial position the levers (420) of the arms extend perpendicular to the nose (40) and the stops (424) extend parallel to the nose (40).

2. The needle holder according to claim 1, **characterized in that** the arms (42) are mirror-symmetrically arranged in relation to the nose (40).

3. The needle holder according to claim 1 or 2, **characterized in that** the nose (40) is provided on a rib (44) having a slot (46) for receiving the needle.

4. A package for surgical suture material, the package (1) comprising a base member (3) with at least one needle holder (4) according to any one of claims 1 to 3.

5. The package according to claim 4, **characterized in that** the nose (40) and the arms (42) are formed integrally with the base member (3).

6. The package according to claim 4 or 5, **characterized in that** the package (1) further comprises a cover member (2), wherein the base member (3) has a plurality of pins (32, 35) protruding from a top surface and the cover member (2) has pin receiving holes (22a, 22b, 23, 25), which pin receiving holes (22a, 22b, 23) are adapted for receiving the pins (32) of the base member (3) for mounting the cover member (2) to the base member (3).

7. The package according to claim 6, **characterized in that** the cover member (2) is provided with a plurality of guiding holes (27), which guiding holes (27) are arranged between the pin receiving holes (22a, 22b, 23) and an outer circumference (21) of the cover member (2) and are adapted for receiving winding pins of a winding apparatus, wherein at least one of the pin receiving holes (22a, 22b) forms a common opening together with a neighboring guiding hole (27).

8. The package according to claim 7, **characterized in that** the pin receiving holes (22a, 22b, 23) and the guiding holes (27) in each case are arranged along an oval path having semi-circular ends, wherein the pin receiving holes (22a, 22b) arranged in the region of the semi-circular ends at least partly form common openings together with the respectively associated neighboring guiding holes (27).

9. The package according to claim 8, **characterized in that** the pin receiving holes (22a, 22b) arranged in the region of the semi-circular ends are at least partly arranged radially inward of the respectively associated neighboring guiding holes (27) of a common opening.

10. The package according to claim 8 or 9, **characterized in that** a radius of a first semi-circular end of the oval path of the pin receiving holes (22a, 22b, 23) is larger than a radius of a second semi-circular end of the oval path of the pin receiving holes (22a, 22b, 23), wherein in particular the pin receiving holes (22b) arranged at the first semi-circular end partly overlap with the associated neighboring guiding holes (27) for forming the common openings and the pin receiving holes (22a) arranged at the second semi-circular end are connected to the associated neighboring guiding holes (27) via bridges (28) for forming the common openings.

11. The package according to claim any one of claims 7 to 10, **characterized in that** the base member (3) is provided with a plurality of guiding holes (37) arranged in alignment with the guiding holes (27) of the cover member (2).

12. The package according to any one of claims 6 to 11, **characterized in that** the pins (32, 35) protruding from a top surface of the base member (3) are arranged along a separation wall (33) protruding from the top surface of the base member (3), which separation wall separates a winding channel (30) of the package from a center area.

13. The package according to claim 12, **characterized in that** the needle holder (4) is provided at the center area, wherein the separation wall (33) has an opening permitting a passage of the suture material.

14. The package according to claim any one of claims 6 to 13, **characterized in that** the base member (3) and the cover member (2) at their outer circumferences are provided with interlocking teeth (310) and notches (210).

15. The package according to any one of claims 6 to 14, **characterized in that** the cover member (2) is made from paper material.

## Patentansprüche

1. Nadelhalter (4) zum Sichern einer Nadel in einer Verpackung für chirurgisches Nahtmaterial, wobei der Nadelhalter (4) eine aus einer Basisebene herausragende Nase (40) und zwei Arme (42), die in einer zur Basisebene parallelen Ebene elastisch verformbar und/oder verschiebbar sind, aufweist, wobei die Arme (42) jeweils mit einem Anschlag (424) zur Anlage an der Nadel versehen sind, wobei die Anschläge (424) an gegenüberliegenden Seiten der Nase (40) angeordnet sind, wobei ein vorderes Ende der Nase (40) als Gegenlager für die Nadel zwischen den Armen (42) und der Nase (40) dient, und wobei die Arme (42) eingerichtet sind, um die von den Anschlägen (424) berührte Nadel gegen das vordere Ende der Nase (40) zu drücken, wobei die Arme (42) L-förmig sind, umfassend einen schwenkbar gelagerten Hebel (420) mit einem ersten, als Drehpunkt dienenden Ende (421) und einem zweiten Ende (422), wobei der Anschlag (424) in einer zur Basisebene parallelen Ebene vom zweiten Ende (422) zur Nase (40) hin vorsteht,
**dadurch gekennzeichnet, dass**
in einer neutralen Ausgangsposition die Hebel (420) der Arme sich senkrecht zur Nase (40) und die Anschläge (424) sich parallel zur Nase (40) erstrecken.

2. Der Nadelhalter nach Anspruch 1, **dadurch gekennzeichnet, dass** die Arme (42) spiegelsymmetrisch zu der Nase (40) angeordnet sind.

3. Der Nadelhalter nach Anspruch 1 oder 2, **dadurch gekennzeichnet, dass** die Nase (40) auf einer Rippe (44) mit einem Schlitz (46) zur Aufnahme der Nadel vorgesehen ist.

4. Verpackung für chirurgisches Nahtmaterial, wobei die Verpackung (1) ein Basiselement (3) mit mindestens einem Nadelhalter (4) nach einem der Ansprüche 1 bis 3 umfasst.

5. Die Verpackung nach Anspruch 4, **dadurch gekennzeichnet, dass** die Nase (40) und die Arme (42) einstückig mit dem Grundkörper (3) ausgebildet sind.

6. Die Verpackung nach Anspruch 4 oder 5, **dadurch gekennzeichnet, dass** die Verpackung (1) weiter ein Abdeckelement (2) umfasst, wobei das Basiselement (3) eine Vielzahl von Stiften (32, 35) aufweist, die von einer Oberseite vorstehen, und das Abdeckelement (2) Stiftaufnahmelöcher (22a, 22b, 23, 25) aufweist, wobei die Stiftaufnahmelöcher (22a, 22b, 23) zum Aufnehmen der Stifte (32) des Basiselements (3) zum Befestigen des Abdeckelements (2) an dem Basiselement (3) angepasst sind.

7. Die Verpackung nach Anspruch 6, **dadurch gekennzeichnet, dass** das Abdeckelement (2) mit einer Vielzahl von Führungslöchern (27) versehen ist, wobei die Führungslöcher (27) zwischen den Stiftaufnahmelöchern (22a, 22b, 23) und einem Außenumfang (21) des Abdeckelements (2) angeordnet sind und zur Aufnahme von Wickelstiften einer Wickelvorrichtung eingerichtet sind, wobei mindestens eines der Stiftaufnahmelöcher (22a, 22b) zusammen mit einem benachbarten Führungsloch (27) eine gemeinsame Öffnung bildet.

8. Die Verpackung nach Anspruch 7, **dadurch gekennzeichnet, dass** die Stiftaufnahmelöcher (22a, 22b, 23) und die Führungslöcher (27) jeweils entlang einer ovalen Bahn mit halbkreisförmigen Enden angeordnet sind, wobei die im Bereich der halbkreisförmigen Enden Stiftaufnahmelöcher (22a, 22b) zusammen mit den jeweils zugehörigen benachbarten Führungslöchern (27) zumindest teilweise gemeinsame Öffnungen bilden.

9. Die Verpackung nach Anspruch 8, **dadurch gekennzeichnet, dass** die Stiftaufnahmelöcher (22a, 22b), die im Bereich der halbkreisförmigen Enden angeordnet sind, zumindest teilweise radial innerhalb der jeweils zugehörigen benachbarten Führungslöcher (27) einer gemeinsamen Öffnung angeordnet sind.

10. Die Verpackung nach Anspruch 8 oder 9, **dadurch gekennzeichnet, dass** ein Radius eines ersten halbkreisförmigen Endes der ovalen Bahn an Stiftaufnahmelöchern (22a, 22b, 23) größer ist als ein Radius eines zweiten halbkreisförmigen Endes der ovalen Bahn an Stiftaufnahmelöchern (22a, 22b, 23), wobei insbesondere die am ersten halbkreisförmigen Ende angeordneten Stiftaufnahmelöcher (22b) die zugehörigen benachbarten Führungslöcher (27) zur Bildung der gemeinsamen Öffnungen teilweise überlappen und die am zweiten halbkreisförmigen Ende angeordneten an Stiftaufnahmelöcher (22a) mit den zugehörigen benachbarten Führungslöchern (27) über Brücken (28) zur Bildung der gemeinsamen Öffnungen verbunden sind

11. Die Verpackung nach einem der Ansprüche 7 bis 10, **dadurch gekennzeichnet, dass** das Basiselement (3) mit einer Vielzahl von Führungslöchern (37) versehen ist, die mit den Führungslöchern (27) des Abdeckelements (2) fluchten.

12. Die Verpackung nach einem der Ansprüche 6 bis 11, **dadurch gekennzeichnet, dass** die Stifte (32, 35), die von einer Oberseite des Basiselements (3) vorstehen, entlang einer Trennwand (33) angeordnet sind, die von der Oberseite des Basiselements (3) vorsteht, wobei die Trennwand einen Wickelkanal (30) der Verpackung von einem Mittelbereich trennt.

13. Die Verpackung nach Anspruch 12, **dadurch gekennzeichnet, dass** der Nadelhalter (4) in dem Mittelbereich vorgesehen ist, wobei die Trennwand (33) eine Öffnung aufweist, die einen Durchgang des Nahtmaterials ermöglicht.

14. Die Verpackung nach einem der Ansprüche 6 bis 13, **dadurch gekennzeichnet, dass** das Basiselement (3) und das Abdeckelement (2) an ihren Außenumfängen mit ineinander greifenden Zähnen (310) und Kerben (210) versehen sind.

15. Die Verpackung nach einem der Ansprüche 6 bis 14, **dadurch gekennzeichnet, dass** das Abdeckelement (2) aus Papiermaterial hergestellt ist.

## Revendications

1. Support d'aiguille (4) pour fixer une aiguille dans un emballage pour matériel de suture chirurgical, le support d'aiguille (4) comportant un élément saillant (40) faisant saillie à partir d'un plan de base, et deux bras (42), qui sont déformables élastiquement et/ou déplaçables dans un plan parallèle au plan de base, les bras (42) étant chacun pourvus d'une butée (424) destinée à entrer en contact avec l'aiguille, les butées (424) étant situées sur des côtés opposés de l'élément saillant (40), une extrémité avant de l'élément saillant (40) servant de contre-portée pour l'aiguille entre les deux butées (424), et les bras (42) étant propres à solliciter l'aiguille, avec laquelle les butées (424) sont en contact, contre l'extrémité avant de l'élément saillant (40), les bras (42) étant en forme de L, comprenant un levier monté pivotant (420) comportant une première extrémité (421) servant d'appui de pivotement, et une seconde extrémité (422), la butée (424) faisant saillie à partir de la seconde extrémité (422) en direction de l'élément saillant (40) dans un plan parallèle au plan de base, **caractérisé en ce que**, dans une position initiale neutre, les leviers (420) des bras s'étendent perpendiculairement à l'élément saillant (40) et les butées (424) s'étendent parallèlement à l'élément saillant (40).

2. Support d'aiguille selon la revendication 1, **caractérisé en ce que** les bras (42) sont disposés en symétrie spéculaire relativement à l'élément saillant (40) .

3. Support d'aiguille selon la revendication 1 ou 2, **caractérisé en ce que** l'élément saillant (40) est placé sur une nervure (44) comportant une fente (46) destinée à recevoir l'aiguille.

4. Emballage pour matériel de suture chirurgical, l'emballage (1) comprenant un élément formant base (3) comportant au moins un support d'aiguille (4) selon l'une quelconque des revendications 1 à 3.

5. Emballage selon la revendication 4, **caractérisé en ce que** l'élément saillant (40) et les bras (42) font partie intégrante de l'élément formant base (3).

6. Emballage selon la revendication 4 ou 5, **caractérisé en ce que** l'emballage (1) comprend, en outre, un élément de recouvrement (2), l'élément formant base (3) comportant une pluralité d'ergots (32, 35) faisant saillie à partir d'une surface supérieure et l'élément de recouvrement (2) comportant des trous de réception d'ergots (22a, 22b, 23, 25), lesdits trous de réception d'ergots (22a, 22b, 23) étant propres à recevoir les ergots (32) de l'élément formant base (3) pour adapter l'élément de recouvrement (2) sur l'élément formant base (3) .

7. Emballage selon la revendication 6, **caractérisé en ce que** l'élément de recouvrement (2) est pourvu d'une pluralité de trous de guidage (27), lesdits trous de guidage (27) étant situés entre les trous de réception d'ergots (22a, 22b, 23) et une circonférence extérieure (21) de l'élément de recouvrement (2) et étant propres à recevoir des ergots de bobinage d'un appareil de bobinage, au moins un des trous de réception d'ergots (22a, 22b) formant une ouverture commune conjointement avec un trou de guidage (27) voisin.

8. Emballage selon la revendication 7, **caractérisé en ce que** les trous de réception d'ergots (22a, 22b, 23) et les trous de guidage (27) sont respectivement situés le long d'un chemin ovale comportant des extrémités semi-circulaires, les trous de réception d'ergots (22a, 22b) situés dans la région des extrémités semi-circulaires formant au moins partiellement des ouvertures communes conjointement avec les trous de guidage (27) voisins respectivement associés.

9. Emballage selon la revendication 8, **caractérisé en ce que** les trous de réception d'ergots (22a, 22b) situés dans la région des extrémités semi-circulaires sont au moins partiellement situés radialement vers l'intérieur relativement aux trous de guidage (27) voisins respectivement associés d'une ouverture commune.

10. Emballage selon la revendication 8 ou 9, **caractérisé en ce qu'**un rayon d'une première extrémité semi-circulaire du chemin ovale des trous de réception d'ergots (22a, 22b, 23) est supérieur à un rayon d'une seconde extrémité semi-circulaire du chemin ovale des trous de réception d'ergots (22a, 22b, 23), en particulier les trous de réception d'ergots (22b) situés au niveau de la première extrémité semi-circulaire étant partiellement superposés aux trous de guidage (27) voisins associés pour former les ouvertures communes et les trous de réception d'ergots (22a) situés au niveau de la seconde extrémité semi-circulaire étant raccordés aux trous de guidage (27) voisins associés par le biais de liaisons (28) pour former les ouvertures communes.

11. Emballage selon l'une quelconque des revendications 7 à 10, **caractérisé en ce que** l'élément formant base (3) est pourvu d'une pluralité de trous de guidage (37) situés dans l'alignement des trous de guidage (27) de l'élément de recouvrement (2).

12. Emballage selon l'une quelconque des revendications 6 à 11, **caractérisé en ce que** les ergots (32, 35) faisant saillie à partir d'une surface supérieure de l'élément formant base (3) sont situés le long d'une paroi de séparation (33) faisant saillie à partir de la surface supérieure de l'élément formant base (3), ladite paroi de séparation formant une séparation entre un canal de bobinage (30) de l'emballage et une région centrale.

13. Emballage selon la revendication 12, **caractérisé en ce que** le support d'aiguille (4) est placé au niveau de la région centrale, la paroi de séparation (33) comportant une ouverture permettant un passage du matériel de suture.

14. Emballage selon l'une quelconque des revendications 6 à 13, **caractérisé en ce que** l'élément formant base (3) et l'élément de recouvrement (2) sont pourvus, au niveau de leurs circonférences extérieures, de dents (310) et de crans (210) propres à s'emboîter.

15. Emballage selon l'une quelconque des revendications 6 à 14, **caractérisé en ce que** l'élément de recouvrement (2) est fait en un matériau de papier.
